# EUROPEAN PATENT APPLICATION

(11) **EP 4 390 945 A1**
(43) Date of publication of application: **26.06.2024**
(21) Application number: 22216198.6
(22) Date of filing: 22.12.2022
(51) Int. Cl.: G16H 10/40, G16H 40/40, G01N 35/00

(54) **MANAGEMENT OF QUALITY CONTROL OF ANALYZING DEVICES CONFIGURED TO PERFORM ANALYSIS OF BIOLOGICAL SAMPLES**

(71) Applicant: Radiometer Medical ApS, 2700 Brønshøj (DK)
(72) Inventor: HAYES, Patrick Vincent, 2700 Brønshøj (DK)
(74) Representative: Inspicos P/S

(57) **Abstract**

A computer-implemented method is disclosed for managing quality control of analyzing devices configured to perform analysis of biological samples. Each of the analyzing devices is configured to perform measurements on biological samples and provide parameter values based on the measurements. Quality control of an analyzing device comprises provision of quality control parameter values by performing measurements on samples of a quality control set, and comparison of the quality control parameter values to acceptance ranges associated with the quality control set.

The method comprises receiving (from one of the analyzing devices) quality control data comprising quality control results associated with a quality control set identifier, and evaluating the received quality control results in relation to requirements for quality control set validation responsive to an initial reception of quality control data for the quality control set identifier. Responsive to the received quality control results passing the evaluation, the method comprises storing acceptance ranges for the quality control set in association with the quality control set identifier, for further quality controls with the quality control set identifier.

Corresponding computer program product, computer-based data processing system, server node, arrangement, and use are also disclosed.

## Description

### TECHNICAL FIELD

The present disclosure relates generally to the field of analyzing devices configured to perform analysis of biological samples. More particularly, it relates to management of quality control of such devices.

### BACKGROUND

Analyzing devices configured to perform analysis of biological samples are useful, for example, in the medical/clinical field. For example, a biological sample from a patient may be inserted into an analyzing device via a sample input, and a result of the analysis may be provided via an operator interface of the analyzing device (e.g., a screen) to form a basis for medical considerations.

Other contexts where analyzing devices configured to perform analysis of biological samples may be useful include laboratory work, the food industry, and various types of research scenarios.

Generally, when an analyzing device is referred to herein, it is meant to encompass any electronic device suitable for performing analysis of biological samples. The analysis of the biological sample may be any suitable analysis (e.g., an analysis according to the prior art). Example analyzing devices include medical devices, point-of-care (POC) devices, blood gas analyzers, immunoassay analyzers, etc.

Also generally, when a biological sample is referred to herein, it is meant to encompass any suitable biological material. Example biological samples include biological liquids (e.g., blood, urine, saliva, spinal fluid, etc.) and biological solid materials (e.g., tissue biopsy, etc.). Typically, the biological sample is carried by a sample container (e.g., a blood gas syringe, a capillary tube, a test tube, a microscope slide, etc.) when inserted via the sample input.

An analyzing device may be defined as a device configured to perform measurements on biological samples and provide parameter values based on the measurements. Generally, the parameter values may be values of any suitable parameter(s) (e.g., parameter values provided by analyses according to the prior art). Example parameters and parameter values include pH (acidity/alkalinity), hydrogen ion concentration, *p*CO₂(a) (a measure of the amount of CO₂ dissolved in blood), actual bicarbonate concentration, standard bicarbonate concentration (actual bicarbonate concentration corrected for abnormal *p*CO₂(a) concentration), base excess (amount of base that needs to be added to, or subtracted from, blood to achieve a normal pH - typically 7.40 - after correction for abnormal *p*CO₂(a) concentration), *p*O₂ (a measure of the amount of oxygen dissolved in blood), *s*O₂ (oxygen saturation; percentage of total hemoglobin oxygen binding sites available in blood that are actually occupied by oxygen), hemoglobin concentration, protein substances, cholesterol, etc.

To ensure that an analyzing device is working properly, and is safe and effective, quality controls of the device should typically be performed.

Typically, quality control of an analyzing device comprises provision of quality control parameter values by performing measurements on samples (a.k.a., quality control, QC, samples) of a quality control set (a.k.a., a quality control, QC, lot). The quality control set may comprise a collection of (preferably un-tamperable) sample containers with respective QC samples, wherein each of the QC samples have known parameter values.

Generally, the QC samples of a quality control set may be biological samples, or samples of nonbiological origin. For example, a QC sample may be defined as a control substance, or a QC specimen, having a measurable property that is carefully controlled, such that when an analyzing device performs a measurement on the QC sample to verify its operational accuracy, there is an associated acceptance range (e.g., a target range comprising a target value ± a tolerance value) that the measurement results should fall within in order to fulfil the requirements for quality control.

Also generally, a QC lot may comprise any suitable collection of QC samples. For example, a QC lot may comprise QC samples in the form of liquids contained in respective sample containers (e.g., pouches, and/or ampules, and/or bottles) configured to be connected to the analyzer device for QC measurements.

The quality control set may be provided by a manufacturer of the analyzing device, by a regulatory organization, or by any other suitable actor.

The quality control set is typically associated with acceptance ranges for the parameter values. Each QC sample of the quality control set may be associated with a respective acceptance range for each of one or more parameter value(s), or a group of QC samples of the quality control set may be associated with a collective acceptance range for each of one or more parameter value(s). For example, a QC sample of the quality control set may be associated with a pH acceptance range defining that the measured pH should be between, e.g., 7.35 and 7.45.

The acceptance ranges may be provided together with the quality control set. Alternatively or additionally, the acceptance ranges may be provided by a manufacturer of the analyzing device, a regulatory organization, or by the organization operating the analyzing device (e.g., a hospital organization). For example, a regulatory organization or the organization operating the analyzing device may apply acceptance ranges which are stricter (i.e., narrower) than those provided together with the quality control set.

Quality control of an analyzing device comprises comparison of the quality control parameter values (which are provided by measurements on QC samples of the quality control set) to corresponding acceptance ranges. If a quality control parameter value is within the corresponding acceptance range, the analyzing device may be considered to have completed the related measurement adequately. If a quality control parameter value is not within the corresponding acceptance range, the related measurement by the analyzing device may be considered to be in-adequate.

Typically, a quality control occasion for an analyzing device encompasses performing measurements on several QC samples of a quality control set, possibly for several parameters, and comparing the quality control parameter values to the corresponding acceptance ranges. Typically, the analyzing device may be considered to have passed the quality control when a condition for correct measurements is fulfilled (e.g., at least a number of x consecutively correct measurements, at least a number of y measurements of which at least a certain percentage are correct, etc.).

A problem in relation to the quality controls is that whenever a new quality control set is used at an analyzing device, information (e.g., regarding the acceptance ranges) has to be input to the analyzing device by an operator (e.g., by scanning barcodes, by manual typing, etc.). This is typically also the case whenever the information (e.g., the acceptance ranges) has been updated for some reason. To this end, it should be noted that an organization may be operating a relatively large amount of analyzing devices.

Another problem in relation to the quality controls is that whenever a new quality control set is to be used, it typically needs to be validated first. The validation may comprise any suitable validation control relating to the quality control set (e.g., validation according to the prior art).

For example, the validation may comprise performing (a relatively large number of) measurements on QC samples of a quality control set using a trusted analyzing device (e.g., an analyzing device which has recently passed a quality control using another quality control set), and evaluating the results in relation to requirements for quality control set validation. For example, the requirements may include that all (or at least some percentage of) the quality control parameter values resulting from the measurements are within the corresponding acceptance ranges. Alternatively or additionally, the requirements may include conditions for statistical metrics (e.g., mean, variance, covariance, standard deviation, etc.) of the quality control parameter values resulting from the measurements.

An example set of requirements for quality control set validation may comprise that a specified number of measurements needs to be performed on a specified number of analyzing devices and over a specified period of time, and that a resulting statistical value (e.g., variance, covariance, standard deviation) needs to be lower than, or equal to, a threshold value.

Inputting information, such as the acceptance ranges, to the analyzing device(s) may be a timeconsuming and/or error-prone task, as well as the task of validating a quality control set.

Therefore, there is a need for alternative approaches to management of quality control for analyzing devices configured to perform analysis of biological samples.

### SUMMARY

It should be emphasized that the term "comprises/comprising" (replaceable by "includes/including") when used in this specification is taken to specify the presence of stated features, integers, steps, or components, but does not preclude the presence or addition of one or more other features, integers, steps, components, or groups thereof. As used herein, the singular forms "a", "an" and "the" are intended to include the plural forms as well, unless the context clearly indicates otherwise.

Generally, when an arrangement is referred to herein, it is to be understood as a physical product; e.g., an apparatus. The physical product may comprise one or more parts, such as controlling circuitry in the form of one or more controllers, one or more processors, or the like.

It is an object of some embodiments to solve or mitigate, alleviate, or eliminate at least some of the above or other disadvantages.

A first aspect is a computer-implemented method for managing quality control of analyzing devices configured to perform analysis of biological samples. Each of the analyzing devices is configured to perform measurements on biological samples and provide parameter values based on the measurements. Quality control of an analyzing device comprises provision of quality control parameter values by performing measurements on samples of a quality control set, and comparison of the quality control parameter values to acceptance ranges associated with the quality control set.

The method comprises receiving (from one of the analyzing devices) quality control data comprising quality control results associated with a quality control set identifier, and - responsive to an initial reception of quality control data for the quality control set identifier - evaluating the received quality control results in relation to requirements for quality control set validation. The method also comprises - responsive to the received quality control results passing the evaluation - storing acceptance ranges for the quality control set in association with the quality control set identifier, for further quality controls with the quality control set identifier.

In some embodiments, the method further comprises storing an evaluation result indication in association with the quality control set identifier, wherein the evaluation indication comprises a pass indication or a fail indication.

In some embodiments, the acceptance ranges are comprised in the quality control data for the initial reception of the quality control set identifier.

In some embodiments, the method further comprises receiving the acceptance ranges from a remote quality control update service.

In some embodiments, the method further comprises generating an update-indicating administration notification responsive to receiving the acceptance ranges from the remote quality control update service.

In some embodiments, the method further comprises providing the stored acceptance ranges to one or more of the analyzing devices for further quality controls with the quality control set identifier.

In some embodiments, the method further comprises - responsive to the received quality control results failing the evaluation - generating a failure-indicating administration notification.

In some embodiments, the method further comprises generating a warning notification responsive to reception of further quality control data comprising the quality control set identifier.

In some embodiments, the method further comprises receiving a re-evaluation input for the quality control set identifier, wherein the re-evaluation input indicates a result of an additional evaluation of the received quality control results in relation to the requirements for quality control set validation.

In some embodiments, the method further comprises - responsive to the re-evaluation input indicating that the received quality control results passed the additional evaluation - storing acceptance ranges for the quality control set in association with the quality control set identifier, for further quality controls with the quality control set identifier.

In some embodiments, the method further comprises storing a re-evaluation result indication in association with the quality control set identifier, wherein the re-evaluation indication comprises a pass indication or a fail indication.

A second aspect is a computer program product comprising a non-transitory computer readable medium, having thereon a computer program comprising program instructions. The computer program is loadable into a data processing unit and configured to cause execution of the method according to the first aspect when the computer program is run by the data processing unit.

A third aspect is a computer-based data processing system for management of quality control of analyzing devices configured to perform analysis of biological samples. Each of the analyzing devices is configured to perform measurements on biological samples and provide parameter values based on the measurements. Quality control of an analyzing device comprises provision of quality control parameter values by performing measurements on samples of a quality control set, and comparison of the quality control parameter values to acceptance ranges associated with the quality control set.

The system comprises a device interface for reception (from one of the analyzing devices) of quality control data comprising quality control results associated with a quality control set identifier.

The system also comprises controlling circuitry configured to - responsive to an initial reception of quality control data for the quality control set identifier - evaluate the received quality control results in relation to requirements for quality control set validation, and (responsive to the received quality control results passing the evaluation) cause storing of acceptance ranges for the quality control set in association with the quality control set identifier, for further quality controls with the quality control set identifier.

A fourth aspect is a server node comprising the system of the third aspect.

A fifth aspect is an arrangement for data coordination. The arrangement comprises the system of the third aspect and one or more analyzing devices configured to perform analysis of biological samples.

A sixth aspect is use of the arrangement of the fifth aspect for management of quality control of the one or more analyzing devices configured to perform analysis of biological samples.

In some embodiments, any of the above aspects may additionally have features identical with or corresponding to any of the various features as explained above for any of the other aspects.

An advantage of some embodiments is that alternative approaches are provided for management of quality control for analyzing devices configured to perform analysis of biological samples.

An advantage of some embodiments is that the amount of information that need to be input to analyzing devices (e.g., information of acceptance ranges) is reduced compared to other approaches.

An advantage of some embodiments is that quality control of analyzing devices becomes more time-efficient compared to other approaches.

An advantage of some embodiments is that the probability of errors for the acceptance ranges is reduced compared to other approaches.

An advantage of some embodiments is that the quality control set validation becomes more time-efficient compared to other approaches.

An advantage of some embodiments is that the probability of errors for the quality control set validation is reduced compared to other approaches.

An advantage of some embodiments is that (remote) updating of the acceptance ranges is facilitated compared to other approaches.

### BRIEF DESCRIPTION OF THE DRAWINGS

Further objects, features and advantages will appear from the following detailed description of embodiments, with reference being made to the accompanying drawings. The drawings are not necessarily to scale, emphasis instead being placed upon illustrating the example embodiments.
Figure 1 is a schematic drawing illustrating an example arrangement for data coordination according to some embodiments;
Figure 2 is a schematic block diagram illustrating an example computer-based data processing system according to some embodiments;
Figure 3A is a flowchart illustrating example method steps according to some embodiments;
Figure 3B is a flowchart illustrating example method steps according to some embodiments;
Figure 4A is a signaling diagram illustrating example signaling according to some embodiments;
Figure 4B is a signaling diagram illustrating example signaling according to some embodiments;
Figure 5A is a schematic drawing illustrating an example database entry according to some embodiments;
Figure 5B is a schematic drawing illustrating an example database entry according to some embodiments; and
Figure 6 is a schematic drawing illustrating an example computer readable medium according to some embodiments.

### DETAILED DESCRIPTION

As already mentioned above, it should be emphasized that the term "comprises/comprising" (replaceable by "includes/including") when used in this specification is taken to specify the presence of stated features, integers, steps, or components, but does not preclude the presence or addition of one or more other features, integers, steps, components, or groups thereof. As used herein, the singular forms "a", "an" and "the" are intended to include the plural forms as well, unless the context clearly indicates otherwise.

Embodiments of the present disclosure will be described and exemplified more fully hereinafter with reference to the accompanying drawings. The solutions disclosed herein can, however, be realized in many different forms and should not be construed as being limited to the embodiments set forth herein.

In the following, alternative approaches will be provided for management of quality control for analyzing devices configured to perform analysis of biological samples.

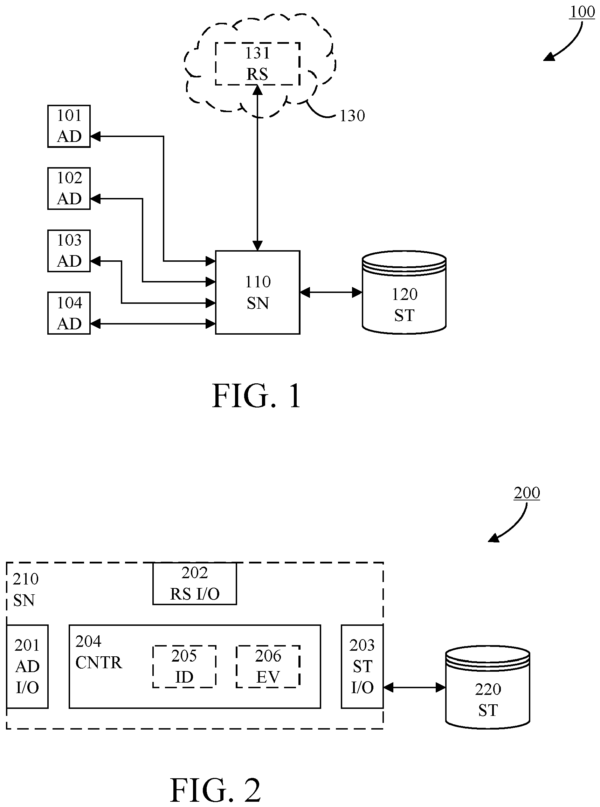

Figure 1 schematically illustrates an example arrangement 100 for data coordination according to some embodiments.

The arrangement 100 comprises a plurality of analyzing devices (AD) 101, 102, 103, 104 configured to perform analysis of biological samples. For example, the analyzing devices 101, 102, 103, 104 may be located within an organization, such as a hospital, or a group of hospitals. Each of the analyzing devices 101, 102, 103, 104 is configured to perform measurements on biological samples and provide parameter values based on the measurements.

The arrangement 100 may be used for management of quality control of the analyzing devices 101, 102, 103, 104. Quality control of an analyzing device 101, 102, 103, 104 comprises provision of quality control parameter values by performing measurements on (e.g., biological) samples of a quality control set, and comparison of the quality control parameter values to acceptance ranges associated with the quality control set.

The arrangement 100 also comprises a server node (SN) 110. The server node 110 is operatively connected to at least some of the analyzing devices 101, 102, 103, 104 (e.g., via wired, or wireless, communication interfaces). For example, the server node 110 may be located within the same organization as the analyzing devices 101, 102, 103, 104 (e.g., in the form of a server - or data center - on premise, forming part of a hospital organization infrastructure). Alternatively, the server node 110 may be located external to the organization of the analyzing devices 101, 102, 103, 104 (e.g., in the form of a cloud server).

The arrangement 100 further comprises storage (ST) 120. The storage 120 is operatively connected to the server node 110. The storage 120 may be comprised in the server node 110, or may be external to the server node 110. The storage 120 may be any suitable form of data storage (e.g., data storage according to the prior art). For example, the storage 120 may be located within the same organization as the analyzing devices 101, 102, 103, 104 (e.g., in the form of a storage on premise, forming part of a hospital organization infrastructure). Alternatively, the storage 120 may be located external to the organization of the analyzing devices 101, 102, 103, 104 (e.g., in the form of cloud storage).

In some embodiments, the arrangement 100 may comprise, or be otherwise associated with (e.g., operatively connected, or connectable, to), a remote server (RS) 131. Typically, the remote server 131 is a cloud server, as indicated by 130.

Figure 2 schematically illustrates an example computer-based data processing system 200 according to some embodiments. For example, the system 200 may be comprised in the arrangement 100 of Figure 1. The system 200 is for management of quality control of analyzing devices (AD) configured to perform analysis of biological samples (compare with the analyzing devices 101, 102, 103, 104 of Figure 1).

The system 200 comprises a device interface (AD I/O) 201 for communication with the analyzing devices (AD). The system 200 may also comprise a storage interface (ST I/O) 203 for communication with storage (ST) 220 (compare with storage 120 of Figure 1). In some embodiments, the system 200 further comprises a remote server interface (RS I/O) 202 for communication with a remote server (RS) (compare with the remote server 131 of Figure 1). The interface(s) may, for example, comprise communication interfaces (e.g., connection port(s) for wired communication, or wireless communication transceiver(s)).

The device interface 201 is configured for reception, from one of the analyzing devices, of quality control data comprising quality control results associated with a quality control set identifier. The quality control results typically comprises quality control parameter values provided by the analyzing device by performing measurements on QC samples of a quality control set. Alternatively or additionally, the quality control results may comprise a result (e.g., measurements adequate/in-adequate) of the comparison of the quality control parameter values to acceptance ranges associated with the quality control set.

The system 200 also comprises a controller (CNTR; e.g., controlling circuitry or a control module) 204. The interface(s) 201, 202, 203 and the controller 204 may be comprised in a server node (SN) 210 according to some embodiments (compare with the server node 110 of Figure 1).

The controller 204 is configured to determine whether or not the quality control set identifier has been previously received (i.e., whether or not the quality control set has been used before). To this end, the controller 204 may comprise, or be otherwise associated with (e.g., connected, or connectable, to), an identifier determiner (ID; e.g., identifier determining circuitry or an identifier determination module) 205. The identifier determiner 205 may be configured to determine whether or not the quality control set identifier has been previously received. For example, the controller 204 may be configured to check whether or not there is any record of previous reception of the quality control set identifier in the storage 220.

Responsive to an initial reception of quality control data for the quality control set identifier (i.e., when the quality control set identifier has not been previously received), the controller 204 is configured to evaluate the received quality control results in relation to requirements for quality control set validation. To this end, the controller 204 may comprise, or be otherwise associated with (e.g., connected, or connectable, to), an evaluator (EV; e.g., evaluating circuitry or an evaluation module) 206. The evaluator 206 may be configured to evaluate the received quality control results in relation to the requirements for quality control set validation.

Responsive to the received quality control results passing the evaluation, the controller 204 is configured to cause storing (e.g., in the storage 220) of acceptance ranges for the quality control set in association with the quality control set identifier.

The stored acceptance ranges are to be used for further quality controls with the quality control set identifier. The acceptance ranges may be provided to all relevant analyzing devices via the device interface 201. For example, the acceptance ranges may be pushed to all relevant analyzing devices in association with the storing. Alternatively or additionally, the acceptance ranges may be provided to a relevant analyzing device responsive to an explicit or implicit request from the analyzing device. Thereby, the acceptance ranges associated with a quality control set identifier need only be input the first time the quality control set is used on any analyzing device connected to the system 200.

The analyzing devices that are relevant for the acceptance ranges may be grouped and/or kept track of by the system 200 (e.g., by entries in the storage 220). For example, acceptance ranges for a specific quality control set may be applicable (only) for analyzing devices of a particular type and/or version. Alternatively or additionally, acceptance ranges for a specific quality control set may be applicable (only) for analyzing devices from a particular manufacturer. Yet alternatively or additionally, acceptance ranges for a specific quality control set may be applicable (only) for analyzing devices of a particular department, or hospital, or hospital organization. Yet alternatively or additionally, acceptance ranges for a specific quality control set may be applicable (only) for analyzing devices of (e.g., operating in) a particular country, or other type of jurisdiction.

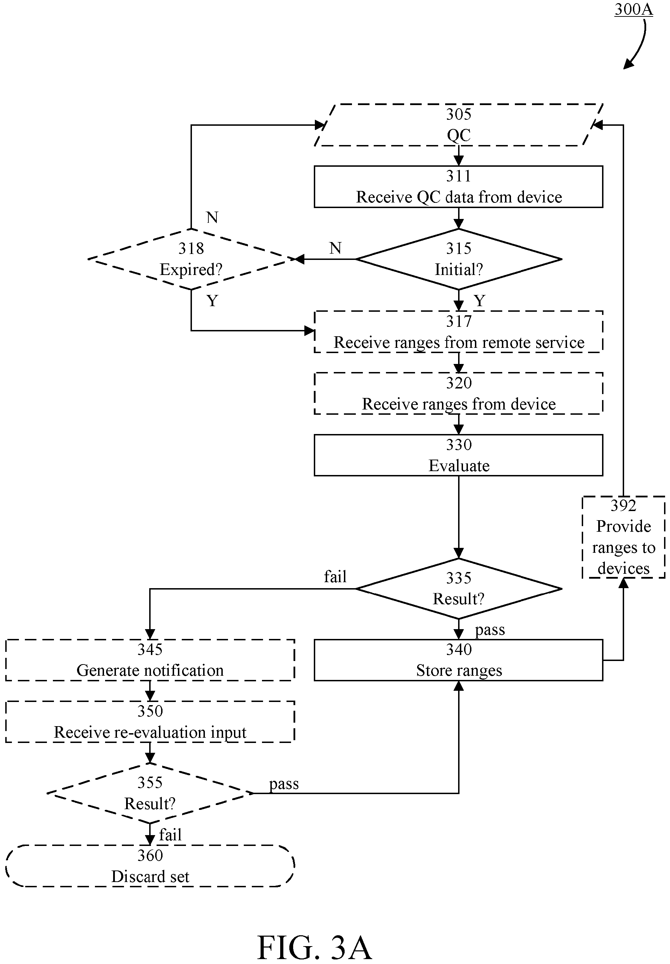

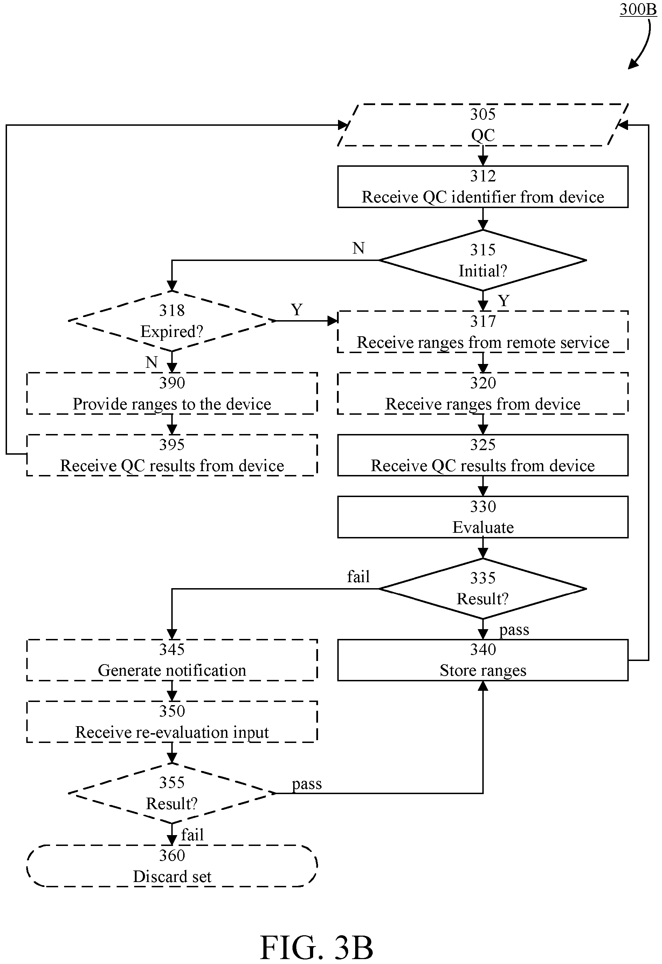

Further possible features of the system 200 will be elaborated on with reference to Figures 3A and 3B, which illustrates a respective example methods 300A and 300B according to some embodiments. For example, the arrangement 100 of Figure 1 and/or the system 200 of Figure 2 may be configured to perform one or more steps of the methods 300A and/or 300B.

The methods 300A and 300B are computer-implemented methods for managing quality control of analyzing devices configured to perform analysis of biological samples, wherein each of the analyzing devices is configured to perform measurements on biological samples and provide parameter values based on the measurements, and wherein quality control of an analyzing device comprises provision of quality control parameter values by performing measurements on QC samples of a quality control set, and comparison of the quality control parameter values to acceptance ranges associated with the quality control set.

The method 300A may be initiated when quality control (QC) measurements are performed for a quality control set at an analyzing device, as indicated by optional step 305.

In step 311, quality control data is received from the analyzing device (e.g., via a device interface 201). The quality control data comprises quality control results (quality control parameter values as measured by the analyzing device and/or information regarding whether the measurements where adequate or in-adequate based on the comparison with the acceptance ranges) associated with a quality control set identifier.

In step 315, it is determined whether or not the quality control set identifier has been previously received. When the quality control set identifier has not been previously received - i.e., when it is an initial reception of quality control data for the quality control set identifier (Y-path out of step 315) - the method 300A comprises evaluating the received quality control results in relation to requirements for quality control set validation (e.g., perform quality control set validation based on the received quality control results and the requirements), as illustrated by step 330.

When the received quality control results passes the evaluation ("pass"-path out of step 335), the method 300A comprises storing the storing acceptance ranges for the quality control set in association with the quality control set identifier, as illustrated by step 340.

The acceptance ranges to be stored may be received from the analyzing device (where they have been input by an operator at the initial use of the quality control set), as illustrated by optional step 320. In some embodiments, the acceptance ranges to be stored are received from the analyzing device in step 311; as part of the quality control data.

Alternatively or additionally, the acceptance ranges to be stored may be received from a remote quality control update service (e.g., via a remote server interface 202), as illustrated by optional step 317. The remote quality control update service may be a cloud-based service. For example, the remote quality control update service may be provided by a provider of the quality control set, by a manufacturer of the analyzing device, by a regulatory organization, or by any other suitable actor.

The stored acceptance ranges are for further quality controls with the quality control set identifier. To this end, the method 300A may comprise providing the acceptance ranges to all relevant analyzing devices, as illustrated by optional step 392. The provision of the acceptance ranges to the relevant analyzing devices may be performed in association with the acceptance ranges being stored in step 340, or at some later point in time. In some embodiments, the acceptance ranges are provided to a relevant analyzing device on request.

Generally, quality controls of the device should typically be performed repeatedly (e.g., at some predefined time intervals and/or event-triggered).

In relation to a further quality control with the quality control set identifier - i.e., when the quality control set identifier has been previously received and it is not an initial reception of quality control data for the quality control set identifier (N-path out of step 315) - the method 300A typically need not perform any evaluation. However, in some embodiments, the evaluation of step 330 may have time-limited validity, in which case the method 300A may repeat step 330 (e.g., periodically and/or event-triggered) for some further quality controls with a previously received quality control set identifier. This is illustrated by optional step 318, wherein the method 300A proceeds to the evaluation when the time-limited validity has expired (Y-path out of 318) and awaits the next quality control when the time-limited validity has not yet expired (N-path out of 318).

When the received quality control results fails the evaluation ("fail"-path out of step 335), the method 300A may comprise generating a failure-indicating administration notification, as illustrated by optional step 345. Such a notification may be suitable for guiding an administrator of the system to perform one or more corresponding tasks. For example, the administrator may be prompted to prevent the quality control set from being used until a re-evaluation has been performed (e.g., by physically restricting access to the QC samples of the quality control set) and/or to trigger/perform a re-evaluation. The failure-indicating administration notification may be rendered via a central user interface (e.g., associated with an access to the server node).

Alternatively or additionally, when the received quality control results fails the evaluation, the method may comprise generating a warning notification responsive to reception of further quality control data comprising the quality control set identifier (e.g., generating a warning notification if the quality control set is used again before being re-evaluated). Such a notification may be suitable for guiding an operator of the analyzing device to perform one or more corresponding tasks. For example, the operator may be prompted to select another quality control set. In some embodiments, the warning notification may indicate a suitable quality control set (i.e., one that has passed evaluation). The warning notification may be rendered via a user interface at the relevant analyzing device and/or via a central user interface (e.g., associated with an access to the server node).

Alternatively or additionally, when the received quality control results fails the evaluation, the method 300A may comprise receiving a re-evaluation input for the quality control set identifier, as illustrated by optional step 350. The re-evaluation input indicates a result of an additional evaluation of the received quality control results in relation to the requirements for quality control set validation. In some embodiments, the additional evaluation may be performed similarly as has been described for the initial evaluation (compare with step 330); possibly based on new quality control measurements (using other QC samples and/or another analyzing device). In some embodiments, the additional evaluation may be performed manually by an administrator of the system.

When the re-evaluation input indicates that the received quality control results passed the additional evaluation ("pass"-path out of optional step 355), the method 300A proceeds to step 340 for storing of acceptance ranges for the quality control set in association with the quality control set identifier.

When the re-evaluation input indicates that the received quality control results failed the additional evaluation ("fail"-path out of optional step 355), the quality control set might be discarded, as illustrated by optional step 360. For example, step 360 may comprise removing any information regarding the quality control set associated with the quality set identifier from the data storage, or marking the quality control set associated with the quality set identifier as discarded in the data storage. Alternatively or additionally, step 360 may comprise generating a discard-indicating administration notification. Such a notification may be suitable for guiding an administrator of the system to perform one or more corresponding tasks. For example, the administrator may be prompted to prevent any further use of the quality control set (e.g., by physically restricting access to the QC samples of the quality control set or by physically discarding the QC samples of the quality control set). The discard-indicating administration notification may be rendered via a central user interface (e.g., associated with an access to the server node).

According to some embodiments, an indication of the evaluation result of step 335 and/or the re-evaluation result of step 355 may be stored in association with the quality control set identifier. The indication may be a pass indication or a fail indication. In some embodiments, there is one entry for the indication of the evaluation result and one entry for the indication of the re-evaluation result. In some embodiments, there is a single entry for indicating the evaluation result and the re-evaluation result (e.g., the re-evaluation indication over-writing the evaluation indication).

In some embodiments, only pass indications are saved (e.g., having no entry for a quality control set identifier until it has passed evaluation). In some embodiments, only fail indications are saved (e.g., interpreting stored acceptance ranges as an implicit pass indication). In some embodiments, both pass and fail indications are saved.

Generally, updates of the acceptance ranges may be received from the remote quality control update service at any time. For example, the acceptance ranges may be pushed from the remote quality control update service when there is an update of the ranges. Alternatively or additionally, the acceptance ranges may be provided from the remote quality control update service on request. According to some embodiments, the method may comprise querying the remote quality control update service for acceptance range updates at predetermined times (e.g., periodically) and/or event-based (e.g., responsive to one or more of the following triggers: when quality control data is received from an analyzing device, when the quality control set evaluation fails, when information is missing regarding one or more acceptance ranges, when measurements of an analyzing device are largely determined as in-adequate, etc.).

If updated acceptance ranges are provided when the quality control set evaluation fails, the evaluation may be repeated with the updated acceptance ranges before establishing whether the evaluation actually resulted in fail or pass.

Similarly, if updated acceptance ranges are provided when measurements of an analyzing device are largely determined as in-adequate, the measurements may be compared with the updated acceptance ranges before establishing whether or not the analyzing device should be considered to provide adequate or in-adequate measurements.

When updates of the acceptance ranges are received from the remote quality control update service, the stored acceptance ranges may be updated accordingly (compare with step 340) and/or the updated acceptance ranges may be provided to all relevant analyzing devices (compare with step 392).

The method may comprise generating an update-indicating administration notification responsive to receiving acceptance ranges from the remote quality control update service. Such a notification may be suitable for guiding an administrator of the system to perform one or more corresponding tasks. For example, the administrator may be prompted to manually update unconnected analyzing devices in relation to the acceptance ranges. Alternatively or additionally, the administrator may be prompted to perform new quality controls for one or more of the analyzing devices, taking into account the updated acceptance ranges.

According to some embodiments, the update-indicating administration notification comprises a respective update indication for each analyzing device that the acceptance ranges are applicable to. The update indication may indicate whether or not the updated ranges have been provided to the analyzing device. For example, all analyzing devices which have been automatically provided by the updates acceptance ranges (compare with step 392) may be marked accordingly, while other analyzing devices that the acceptance ranges are applicable to may be marked as not yet updated. Whenever one of these other analyzing devices is updated (either automatically when it connects to the system, or manually) the update indication may change accordingly. Thereby, the administrator is continuously guided to keep the acceptance ranges updated at all relevant analyzing devices; while no unnecessary effort is spent on automatically updated analyzing devices.

According to some embodiments, the update-indicating administration notification comprises a respective quality control indication for each analyzing device that the acceptance ranges are applicable to. The quality control indication may indicate whether or not the updated ranges have been used to perform quality control of the analyzing device. For example, all relevant analyzing devices may be marked as not yet quality controlled based on the latest update. Whenever the updated ranges have been used to perform quality control of one of these analyzing devices the quality control indication may change accordingly. Thereby, the administrator is continuously guided to keep the quality control up to date with the acceptance ranges at all relevant analyzing devices.

Generally, the update-indicating administration notification may be rendered via a user interface at each relevant analyzing device and/or via a central user interface (e.g., associated with an access to the server node).

Although not explicitly shown in Figure 3A, the method 300A may further comprise checking that the quality control set associated with the quality set identifier is still within its usable time frame. This may be seen as part of the evaluation of step 330, for example.

For example, each quality control set may be associated with an expiry date (i.e., the quality control set is not within its usable time frame when the expiry date has passed). Alternatively or additionally, each quality control set may be associated with a time window of usage, which starts when the quality control set is used for the first time (i.e., the quality control set is not within its usable time frame when the duration of the time window of usage has passed after the first usage of the quality control set).

If the quality control set is not within its usable time frame the evaluation may be considered as failed regardless of parameter statistics. In some embodiments, no re-evaluation is considered in such cases and the "fail"-path out of step 335 leads directly to step 360.

The method 300B is similar to the method 300A of Figure 3A, and the corresponding features will not be repeated.

One difference between the method 300B and the method 300A of Figure 3A is that the quality control data is provided in two portions; first the quality control set identifier (as illustrated by step 312) and then - after determination of whether or not the quality control set identifier has been received previously - the quality control results, as illustrated by step 325 and optional step 395.

Another difference between the method 300B and the method 300A of Figure 3A is that the stored acceptance ranges are provided to a relevant analyzing device in response to determining that the quality control set identifier has been received previously (N-path out of step 315). In some embodiments, step 390 is not executed when the relevant analyzing device has already been provided with the most recently updated acceptance ranges.

The method 300B may have the advantage that the amount of signaling between the system and the analyzing devices is reduced compared to the method 300A, while the average analysis latency may be somewhat longer for the method 300B compared to the method 300A.

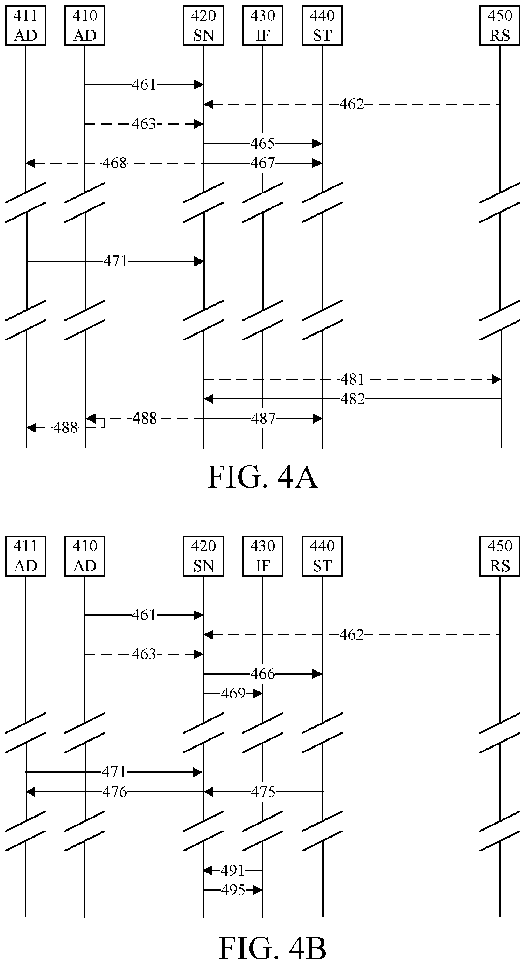

Figure 4A illustrates example signaling among analyzing devices (AD) 410, 411 (compare with 101, 102, 103, 104 of Figure 1), a server node (SN) 420 (compare with 110 of Figure 1 and 210 of Figure 2), a user interface (IF) 430, a data storage (ST) 440 (compare with 120 of Figure 1 and 220 of Figure 2), and a remote server (RS) 450 (compare with 131 of Figure 1).

Quality control data 461 from one of the analyzing devices 410 is received at the server node 420 (compare with step 311 of Figure 3A and steps 312, 325 of Figure 3B).

Acceptance ranges 462, 463 are received at the server node 420 from the analyzing device 410 and/or the remote server 450 (compare with steps 317, 320 of Figures 3A and 3B).

For an initial reception of the quality control set identifier, the quality control set is evaluated (compare with step 330 of Figures 3A and 3B), and when the evaluation is passed, the server node 420 causes a "pass" indication 465 and causes the acceptance ranges 467 to be stored in the data storage 440 (compare with step 340 of Figures 3A and 3B). The acceptance ranges 468 may also be provided (e.g., pushed) to other relevant analyzing devices 411 (compare with step 392 of Figure 3A and step 390 of Figure 3B).

For any further quality controls with the quality control set identifier, the acceptance ranges need not be input (by the operator) to the relevant analyzing device 411 before the quality control data 471 from the analyzing device 411 can be provided to the server node 420.

Updates of the acceptance ranges 482 may be received at the server node 420 from the remote server 450; either pushed autonomously from the remote server, or responsive to a request 481 from the server node 420. The server node 420 causes the updated acceptance ranges 487 to be stored in the data storage 440 and may provide the updated acceptance ranges 488 to the relevant analyzing devices 410, 411.

Figure 4B illustrates example signaling among analyzing devices (AD) 410, 411 (compare with 101, 102, 103, 104 of Figure 1), a server node (SN) 420 (compare with 110 of Figure 1 and 210 of Figure 2), a user interface (IF) 430, a data storage (ST) 440 (compare with 120 of Figure 1 and 220 of Figure 2), and a remote server (RS) 450 (compare with 131 of Figure 1).

Quality control data 461 from one of the analyzing devices 410 is received at the server node 420 (compare with step 311 of Figure 3A and steps 312, 325 of Figure 3B).

Acceptance ranges 462, 463 are received at the server node 420 from the analyzing device 410 and/or the remote server 450 (compare with steps 317, 320 of Figures 3A and 3B).

For an initial reception of the quality control set identifier, the quality control set is evaluated (compare with step 330 of Figures 3A and 3B), and when the evaluation fails, the server node 420 causes a "fail" indication 466 to be stored in the data storage 440 and generates a failure-indicating administration notification 469 (compare with step 345 of Figures 3A and 3B) for rendering via the user interface 430.

For any further quality controls with the quality control set identifier, the server node 420 may - in response to receiving at least the quality control set indicator of the quality control data 471, and after retrieving the "fail" indication 475 from the storage 440 - generate a warning notification 476 for rendering at the relevant analyzing device 411.

A re-evaluation input 491 may be received at the server node 420 via the user interface 430 (compare with step 350 of Figures 3A and 3B).

When the re-evaluation is passed, reference may be made to Figure 4A, where the server node 420 causes a "pass" indication 465 and causes the acceptance ranges 467 to be stored in the data storage 440 (compare with step 340 of Figures 3A and 3B). The acceptance ranges 468 may also be provided (e.g., pushed) to other relevant analyzing devices 411 (compare with step 392 of Figure 3A and step 390 of Figure 3B).

When the re-evaluation fails, the server node 420 generates a discard-indicating administration notification 495 (compare with step 360 of Figures 3A and 3B) for rendering via the user interface 430.

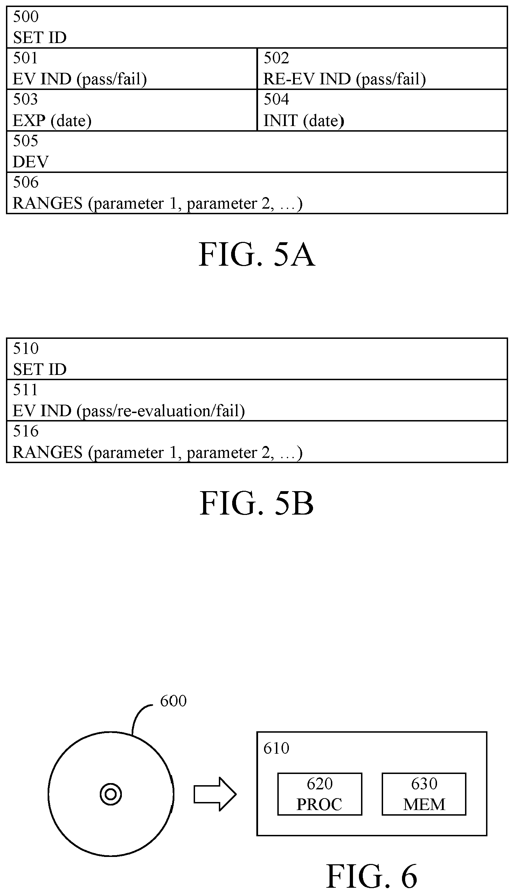

Figure 5A schematically illustrates an example database entry according to some embodiments (e.g., suitable for the storage 120 of Figure 1 and/or the storage 220 of Figure 2). The example database entry relates to a particular quality control set and comprises a field 500 for quality control set identifier (SET ID) and a field 506 for corresponding acceptance ranges (RANGES). The example database entry of Figure 5A also comprises respective fields 501, 502 for evaluation indication (EV IND) and re-evaluation indication (RE-EV IND), as well as respective fields 503, 504 for expiry date (EXP) and initiation time (INIT) for the time window of usage. Furthermore, the example database entry of Figure 5A comprises a field 505 for keeping track of which analyzing devices (DEV) are relevant in relation to the acceptance ranges.

Figure 5B schematically illustrates a more compact example database entry according to some embodiments (e.g., suitable for the storage 120 of Figure 1 and/or the storage 220 of Figure 2). The example database entry relates to a particular quality control set and comprises a field 510 for quality control set identifier (SET ID) and a field 516 for corresponding acceptance ranges (RANGES). The example database entry of Figure 5B also comprises a single field 511 for (re)evaluation indication (EV IND).

It should be understood that various other combination of fields are possible than those exemplified in Figures 5A and 5B to form a suitable database entry for a quality control set. Furthermore, other information associated with a quality control set may also be comprised in such a database entry.

The described embodiments and their equivalents may be realized in software or hardware or a combination thereof. The embodiments may be performed by general purpose circuitry.

Examples of general purpose circuitry include digital signal processors (DSP), central processing units (CPU), co-processor units, field programmable gate arrays (FPGA) and other programmable hardware. Alternatively or additionally, the embodiments may be performed by specialized circuitry, such as application specific integrated circuits (ASIC). The general purpose circuitry and/or the specialized circuitry may, for example, be associated with or comprised in an apparatus such as a server node.

Embodiments may appear within an electronic apparatus (such as a server node) comprising circuitry and/or logic according to any of the embodiments described herein. Alternatively or additionally, an electronic apparatus (such as a server node) may be configured to perform methods according to any of the embodiments described herein.

According to some embodiments, a computer program product comprises a non-transitory computer readable medium such as, for example, a universal serial bus (USB) memory, a plugin card, an embedded drive, or a read only memory (ROM). Figure 6 illustrates an example computer readable medium in the form of a compact disc (CD) ROM 600. The computer readable medium has stored thereon a computer program comprising program instructions. The computer program is loadable into a data processor (PROC; e.g., a data processing unit) 620, which may, for example, be comprised in a server node 610. When loaded into the data processor, the computer program may be stored in a memory (MEM) 630 associated with, or comprised in, the data processor. According to some embodiments, the computer program may, when loaded into, and run by, the data processor, cause execution of method steps according to, for example, any of the methods illustrated in Figures 3A and 3B, or otherwise described herein. Alternatively or additionally, according to some embodiments, the computer program may, when loaded into, and run by, the data processor, cause execution of signaling according to, for example, any of the signaling diagrams illustrated in Figures 4A and 4B, or otherwise described herein.

Generally, all terms used herein are to be interpreted according to their ordinary meaning in the relevant technical field, unless a different meaning is clearly given and/or is implied from the context in which it is used.

Reference has been made herein to various embodiments. However, a person skilled in the art would recognize numerous variations to the described embodiments that would still fall within the scope of the claims.

For example, the method embodiments described herein discloses example methods through steps being performed in a certain order. However, it is recognized that these sequences of events may take place in another order without departing from the scope of the claims. Furthermore, some method steps may be performed in parallel even though they have been described as being performed in sequence. Thus, the steps of any methods disclosed herein do not have to be performed in the exact order disclosed, unless a step is explicitly described as following or preceding another step and/or where it is implicit that a step must follow or precede another step.

In the same manner, it should be noted that in the description of embodiments, the partition of functional blocks into particular units is by no means intended as limiting. Contrarily, these partitions are merely examples. Functional blocks described herein as one unit may be split into two or more units. Furthermore, functional blocks described herein as being implemented as two or more units may be merged into fewer (e.g. a single) unit.

Any feature of any of the embodiments disclosed herein may be applied to any other embodiment, wherever suitable. Likewise, any advantage of any of the embodiments may apply to any other embodiments, and vice versa.

Hence, it should be understood that the details of the described embodiments are merely examples brought forward for illustrative purposes, and that all variations that fall within the scope of the claims are intended to be embraced therein.

## Claims

1. A computer-implemented method for managing quality control of analyzing devices (101, 102, 103, 104) configured to perform analysis of biological samples,
wherein each of the analyzing devices is configured to perform measurements on biological samples and provide parameter values based on the measurements, and
wherein quality control of an analyzing device comprises provision of quality control parameter values by performing measurements on samples of a quality control set, and comparison of the quality control parameter values to acceptance ranges associated with the quality control set,
the method comprising:
receiving (311, 312, 325), from one of the analyzing devices, quality control data comprising quality control results associated with a quality control set identifier; and
responsive (315) to an initial reception of quality control data for the quality control set identifier:
evaluating (330) the received quality control results in relation to requirements for quality control set validation; and
responsive (335) to the received quality control results passing the evaluation, storing (340) acceptance ranges for the quality control set in association with the quality control set identifier, for further quality controls with the quality control set identifier.

2. The method of claim 1, further comprising storing an evaluation result indication in association with the quality control set identifier, wherein the evaluation indication comprises a pass indication or a fail indication.

3. The method of any of claims 1 through 2, wherein the acceptance ranges are comprised in the quality control data for the initial reception of the quality control set identifier.

4. The method of any of claims 1 through 3, further comprising receiving (317) the acceptance ranges from a remote quality control update service.

5. The method of claim 4, further comprising generating an update-indicating administration notification responsive to receiving the acceptance ranges from the remote quality control update service.

6. The method of any of claims 1 through 5, further comprising providing (390, 392) the stored acceptance ranges to one or more of the analyzing devices for further quality controls with the quality control set identifier.

7. The method of any of claims 1 through 6, further comprising, responsive (335) to the received quality control results failing the evaluation, generating (345) a failure-indicating administration notification.

8. The method of claim 7, further comprising generating a warning notification responsive to reception of further quality control data comprising the quality control set identifier.

9. The method of any of claims 7 through 8, further comprising receiving (350) a re-evaluation input for the quality control set identifier, wherein the re-evaluation input indicates a result of an additional evaluation of the received quality control results in relation to the requirements for quality control set validation.

10. The method of claim 9, further comprising, responsive (355) to the re-evaluation input indicating that the received quality control results passed the additional evaluation, storing (340) acceptance ranges for the quality control set in association with the quality control set identifier, for further quality controls with the quality control set identifier.

11. The method of any of claims 9 through 10, further comprising storing a re-evaluation result indication in association with the quality control set identifier, wherein the re-evaluation indication comprises a pass indication or a fail indication.

12. A computer program product comprising a non-transitory computer readable medium (600), having thereon a computer program comprising program instructions, the computer program being loadable into a data processing unit (110, 204, 620) and configured to cause execution of the method according to any of claims 1 through 11 when the computer program is run by the data processing unit.

13. A computer-based data processing system (200) for management of quality control of analyzing devices (101, 102, 103, 104) configured to perform analysis of biological samples,
wherein each of the analyzing devices is configured to perform measurements on biological samples and provide parameter values based on the measurements, and
wherein quality control of an analyzing device comprises provision of quality control parameter values by performing measurements on samples of a quality control set, and comparison of the quality control parameter values to acceptance ranges associated with the quality control set,
the system comprising:
a device interface (201) for reception, from one of the analyzing devices, of quality control data comprising quality control results associated with a quality control set identifier; and
controlling circuitry (204) configured to - responsive to an initial reception of quality control data for the quality control set identifier:
evaluate the received quality control results in relation to requirements for quality control set validation; and
responsive to the received quality control results passing the evaluation, cause storing of acceptance ranges for the quality control set in association with the quality control set identifier, for further quality controls with the quality control set identifier.

14. A server node (110) comprising the system of claim 13.

15. An arrangement (100) for data coordination, the arrangement comprising the system of claim 13 and one or more analyzing devices configured to perform analysis of biological samples.

16. Use of the arrangement of claim 15 for management of quality control of the one or more analyzing devices configured to perform analysis of biological samples.
